# EUROPEAN PATENT SPECIFICATION

(11) **EP 2 163 206 B1**
(45) Date of publication and mention of the grant of the patent: **12.12.2012**
(21) Application number: 08425604.9
(22) Date of filing: 16.09.2008
(51) Int. Cl.: A61B 17/00, A61B 17/128, A61B 1/04

(54) **Surgical clip delivering wireless capsule**
Drahtlose Kapsel zur Abgabe chirurgischer Klammern
Capsule sans fil libérant une pince chirurgicale

(43) Date of publication of application: 17.03.2010
(73) Proprietor: Scuola Superiore di Studi Universitari e di Perfezionamento Sant'Anna, 56125 Pisa (IT); Ovesco Endoscopy GmbH, 72074 Tübingen (DE)
(72) Inventor: Valdastri, Pietro, 57100 Livorno (IT); Quaglia, Claudio, 56025 Pontedera (Pisa) (IT); Menciassi, Arianna, 56025 Pontedera (Pisa) (IT); Dario, Paolo, 57128 Livorno (IT); Ho, Chi-Nghia, 70180 Stuttgart (DE); Anhoeck, Gunnar, 72762 Reutlingen (DE); Schostek, Sebastian, 72070 Tübingen (DE); Rieber, Fabian, 70193 Stuttgart (DE); Schurr, Marc, 72072 Tübingen (DE)
(74) Representative: Brazzini, Silvia

(56) References cited:
- US-A1- 2003 167 000
- US-A1- 2005 043 587
- US-A1- 2005 273 139

## Description

The present invention relates generally to a wireless medical device for compressing body tissue to prevent haemorrhaging, treating of lesion, and marking of suspected tissue area at a surgical site within the gastrointestinal (Gl) tract. More specifically the invention relates to a wireless capsular device for delivering a surgical clip to a selected site of the gastrointestinal tract.

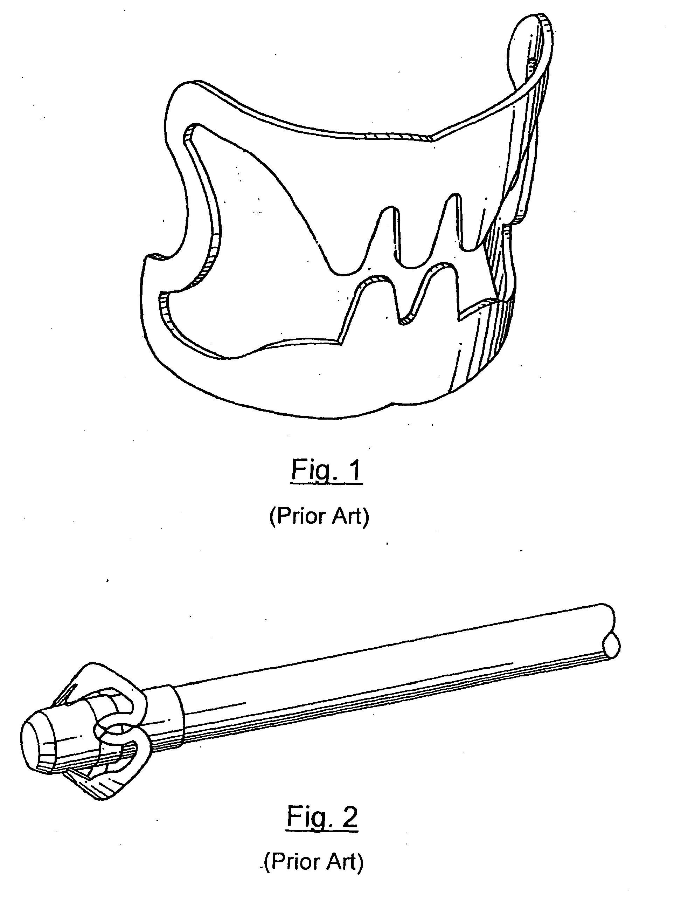

US 6428548 discloses a surgical clip and a system for delivering the clip in a controlled way to a surgical site. This system is intended for use in a variety of medical procedures, including closing an organ perforation from inside a lumen by approximating and compressing the wound edges of the perforated tissue. The system is shown in figures 1 and 2 and comprises an endoscopic device with an endoscope cap at its distal end and a clip removably arranged on the outer surface of the endoscopic cap. The surgical clip is formed by a pair of elastically connected semicircular grasping surfaces, or jaws, having toothed portions facing to each other. When the clip is mounted on the endoscope cap, the clip is in its open, or tissue receiving, position with the toothed portions spaced apart therebetween and elastically forcing on the outer surface of the cap. Once the clip is released under the action of a deployment device, it closes to its tissue grasping position, in which the toothed portions of the jaws are close to each other to grasp the tissue and assist in retaining it between the two yaws.

The two jaws are elastically connected by respective joints that bias the jaws toward each other providing the compression force required to enable the jaws to compress and retain tissue therebetween.

In the use, the surgical clip is deployed off of the endoscope cap after it has been positioned adjacent to the wound site. The deployment device associated with the surgical clip may be of the type including a cable looped around a portion of the clip and actuated by the operator, for pulling forward the clip and deploying it off of the endoscope cap, or of the type including a tubular member movably mounted on the endoscope and actuated by the operator to push the clip from the underside and deploy it off of the endoscope cap.

As any endoscopic procedure, the use of the system for delivering a surgical clip in a controlled way to a surgical site according to US 6428548 entails that a clip dispenser, either a flexible endoscope or another tubular device, be introduced in the patient body through a body orifice and this usually causes serious discomfort and pain to the patient, which is often significantly discouraged to undergo this type of treatment procedures. Furthermore, when using a conventional endoscope, although of the flexible type, it may be very difficult and often impossible to dispense the surgical clip to some segments of the gastrointestinal tract, for example the small bowel.

Wireless endoscopic procedures for painless diagnostic exploration of the gastrointestinal tract are also known. The procedure requires a patient to ingest a vitamin-pill size capsule which is carried by peristalsis through the digestive tract. During the transit the capsule takes images which are transmitted to an array of antennas placed externally at the patient's abdomen and recorded into a portable storage unit attached to a belt around the patient's waist. The acquisition of images takes eight hours and during this time patients are free to conduct their daily activities. The device is expelled naturally after approximately 24 hours, if no complications arise. Then the patient returns to the physician's office to deliver the capsule and the associated equipment to download the images into the physician's workstation for review and analysis.

An endoscopic capsule for wireless endoscopic procedure, as disclosed for instance in US 5604531, comprises a capsule body, a viewing window on the capsule body and a light source such as a LED to illuminate the inner portions of the digestive system, an image sensor to take images and a focusing system, a transmitter, a power source and a data processing unit which generates tracking and video data from the single datastream. A commercial capsule according to this patent is the PillCam® capsule marketed in the United States by InScope, a business division of Johnson & Johnson subsidiary Ethicon Endo-Surgery, for the visualisation of the mucosa of small bowel, colon and esophagus. A similar device is produced by Olympus Corporation and marketed under the name EndoCapsule for the small bowel.

The endoscopic capsules of the above mentioned type move passively through the gastrointestinal tract and thus their movements cannot be controlled. Self propelled and remotely controlled endoscopic capsules have also been disclosed. For example WO2005122866 discloses the application of magnetic forces to permanent magnets in the capsule to move, rotate and stop the capsule in the digestive tract. US2006030754 disclose an active control system for a endoscopic capsule comprising a motor consisting of an electromagnetic stator unit and a permanently magnetized rotor unit, in particular in the form of blades attached to a rotatable axis. The internal power source may receive power from external ultrasonic power sources, electromagnetic wave sources or magnetic sources. EP1715789 discloses an active locomotion system for an endoscopic capsule obtained by means of a set of retractable legs in superelastic SMA housed in axial grooves of the capsule body. Each leg has an active degree of freedom (DOF), to allow rotation of the whole leg around a pivot point to open and close each leg radially, and a passive DOF to bend the leg around an intermediate portion for adapting it to the deformability of the tissue of the gastrointestinal tract.

The endoscopic capsules of the above mentioned type have no therapeutic treatment ability due to the limited space in the capsule. To enable a therapeutic treatment ability the capsule should be equipped with additional mechanical and/or electronical components, which would enlarge the capsule to a unswallowable size. Furthermore, even if the capsule would be maintained to a swallowable size, the energy supply for such a therapeutic mechanism would have to be ensured, which would require more space to be provided for energy storage means.

US 2005/0273139 discloses a swallowable capsule movable in the gastrointestinal tract and carrying a tissue clamping device. The device comprises a clip and a drive unit to provide the clip with an advance and retraction movement toward/from a pre-defined location of the GI tract.

In the capsule according this document the tissue clamping device is arranged inside the capsule and the clip is projected outward from one end of the capsule body after the capsule comes in close proximity to the selected location.

The general object of the present invention is to provide wireless capsular therapeutic treatment ability with lower energy consuming treatment mechanisms.

Another object of the present invention is to provide a wireless capsule capable of moving in the gastrointestinal tract for delivering a surgigal clip to a GI site without the need of tubular endoscopic devices to be introduced in the patient body through a body orifice.

A further object of the present invention is to provide a wireless capsule of the above mentioned type with a surgical clip mounted thereon in such a way to be easily dispensed to a surgical site in the GI tract ensuring an effective tissue compression and tissue capture for the treatment of GI diseases.

A particular object of the present invention is to provide a wireless capsule of the above mentioned type in which both the clip compression strength and the clip releasing action are achieved with a minimum energy demand.

These object are achieved with the wireless capsule for delivering a surgical clip to a surgical site of the Gl tract according to the present invention whose essential features are set forth in claim 1. Further important features are specified in the dependent claims.

According to one aspect of the invention the wireless capsule of the invention comprises a capsule body with a front portion and a surgical clip releasably mounted on the front portion of said capsule body. The capsule body is equipped with wireless locomotion and steering means and image sensing means for taking images of the Gl tract and assisting in leading the capsule up to the target site. A transceiver unit for transmitting the detected images to a data processing unit external to the patient and for receiving control signals from an operator is also provided. Clip retaining and releasing means are provided for retaining the clip on the front portion of the capsule body when the capsule is moved through the Gl tract, the clip retaining and releasing means being wirelessly actuable for releasing the clip when the capsule is in close proximity of the target surgical site and is directed towards it.

In this way the elastic energy stored in the clip once it is mounted on the capsule is exploited both for providing all the compression strength necessary for tissue grasping, without the need of an additional mechanism for generating the compression strength, and for releasing the clip, thus significantly simplifying the releasing mechanism.

According to another aspect of the invention, the wireless capsule is provided with clip retaining and releasing means comprising retractable stoppers, in particular in the form of hooks or pins, projecting from the outer surface of the front portion of the capsule body before the clip. The clip forcibly abuts against the stoppers and a motorized release mechanism is provided inside the capsule body for retracting the stoppers, whereby the clip is released to grasp the tissue at the target site axially facing the capsule.

In a different embodiment of the invention the retaining and releasing means comprise collapsible stoppers, in particular in the form of fluid inflatable bodies or shape memory alloy (SMA) bodies, projecting from the outer surface of the front portion of the capsule body before the clip. The clip forcibly abuts against the stoppers and means are provided inside the capsule body for collapsing the stoppers, in such a way that the clip is released to grasp the tissue at the target site axially facing the capsule.

According to a further aspect of the invention the clip retaining and releasing means comprise pushing means for axially sliding the clip on the outer surface of the front portion of the capsule body until the front edge of the capsule is reached to release the clip. Advantageously, the pushing means may be in the form of a motorized sleeve or ring coaxially movable at the rear side of the clip on the outer surface of the capsule body.

The features and the advantages of the wireless capsule for delivering a surgical clip to a surgical site according to the present invention will be apparent from the following description of exemplifying, non-limiting embodiments thereof made with reference to the accompanying drawings, in which:
Figures 1 and 2 are perspective views of a surgical clip and an endoscopic clip dispenser according to the prior art;
Figure 3 is a side perspective view of the capsule according to the invention;
Figure 4 is a perspective longitudinal sectional view of the capsule of figure 3 showing the releasing device in its clip retaining position;
Figure 5 is a perspective longitudinal sectional view of the capsule showing the releasing device in its clip launching position;
Figure 6 is another perspective longitudinal sectional view of the capsule taken alog lines VI-VI of figure 4 with parts removed for clarity;
Figure 7 is a perspective cross-sectional view of the capsule taken along lines VII-VII of figure 4;
Figure 8 is a perspective longitudinal sectional view of the capsule of the invention showing a first alternative embodiment of the releasing device;
Figures 9a and 9b schematically show a second alternative embodiment of the releasing device in a retaining and launching position respectively;
Figures 10a and 10b schematically show a third alternative embodiment of the releasing device in a retaining and launching position respectively;
Figures 11 a and 11 b schematically show a fourth alternative embodiment of the releasing device in a retaining and launching position respectively;
Figures 12a and 12b schematically show a fifth alternative embodiment of the releasing device in a retaining and launching position respectively;
Figures 13a and 13b schematically show an embodiment of the invention in which means for hooking the tissue at the target area are provided.

As used herein, the word "capsule" means a substantially pill-size elongate cylindrical container with rounded off edges and suitable to be swallowed whole.

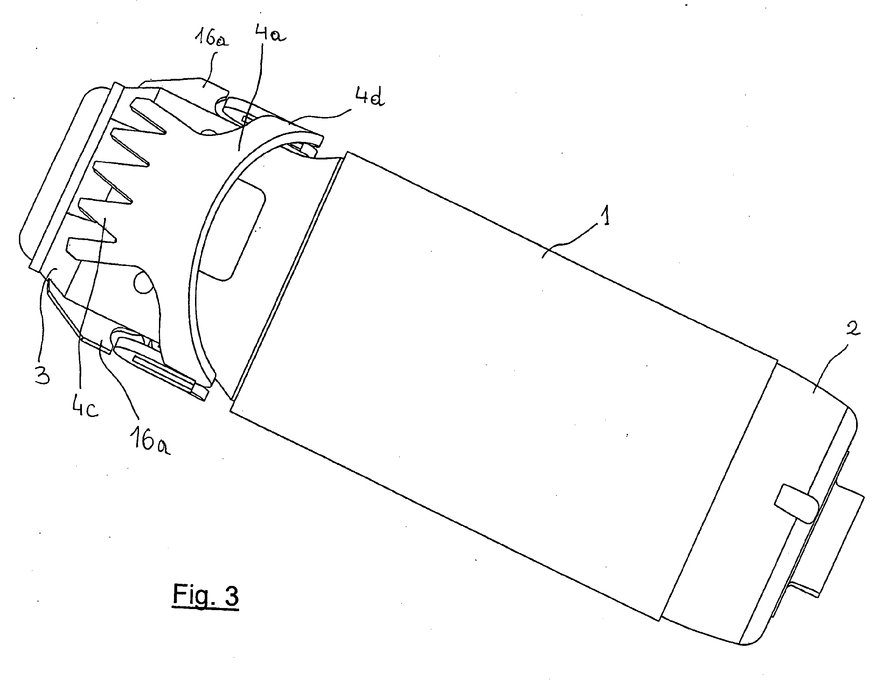

With reference to figure 3 the capsule for delivering a surgical clip at a target area of the gastrointestinal tract according to the invention comprises a capsule body 1 of a substantially cylindrical shape closed at one end with a cap 2. The capsule has a front portion 3 extending from the other end of the capsule body 1 for supporting a surgical clip 4. In particular, the surgical clip 4 is of the known type as disclosed in US 6428548, such as for example the clip shown in figure 1, and is formed by a pair of semicircular tissue grasping surfaces or jaws 4a,b, each having a toothed portion 4c, the jaws being connected to each other at their respective ends by elastic joints 4d.

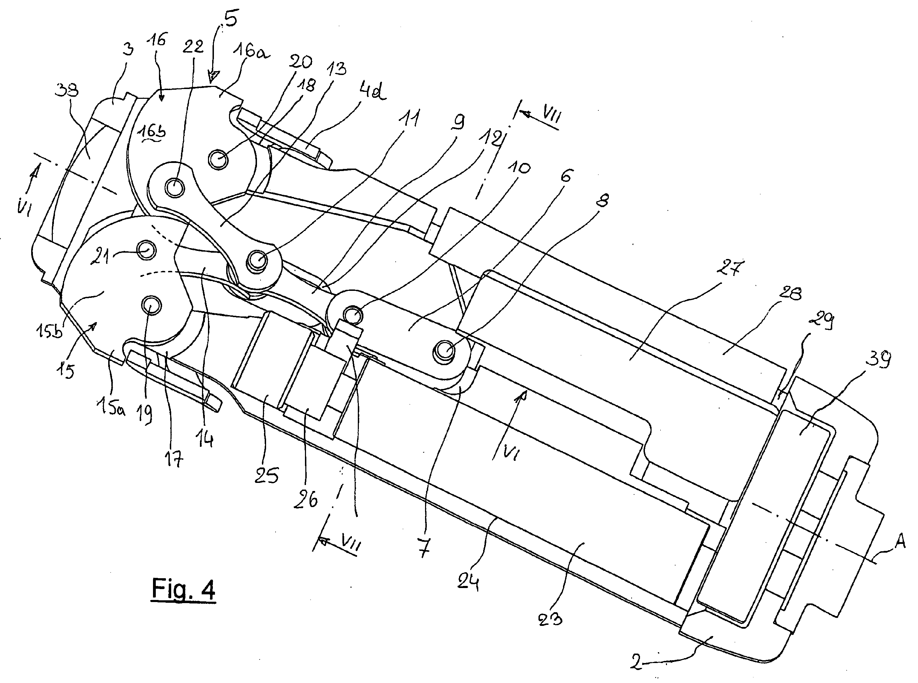

The capsule is equipped with a release device, generally indicated at 5 in figure 4, performing the functions to retain the clip, when the capsule is inoperative and when it is being moved through the gastrointestinal tract and directed toward the target area, and to allow launching the clip once the capsule reaches the target site and its front portion is oriented towards the site.

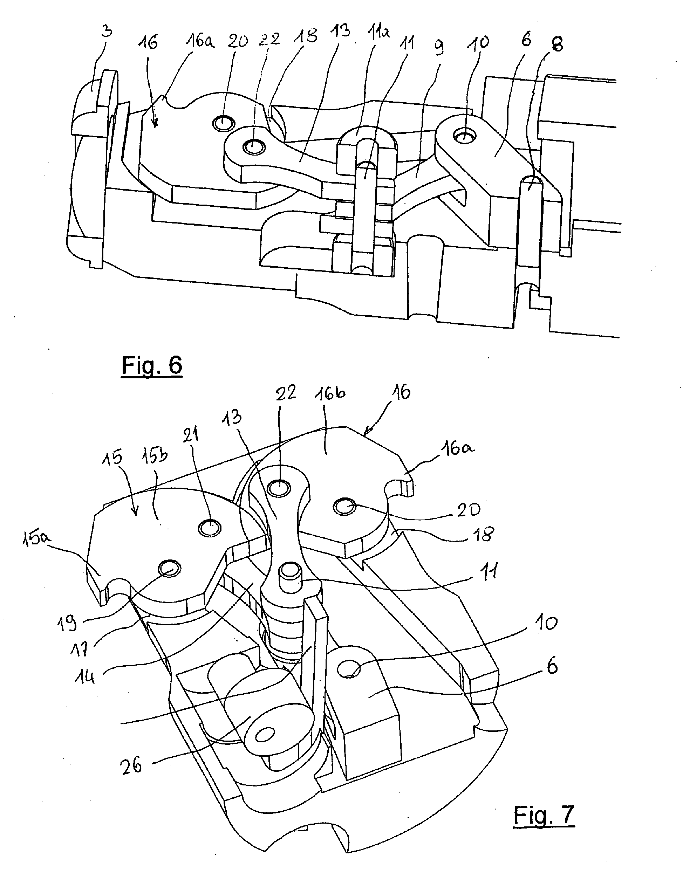

According to the embodiment shown in figures 3 to 7, the release device 5 comprises a first lever 6 housed in a seat 7 in the capsule body 1 and with one end pivotally connected to the seat 7 through a first pin 8 orthogonal to the longitudinal axis A of the capsule. The first lever 6 extends in a substantially axial direction and is pivotally connected to one end of a second lever 9 through a second pin 10, parallel to first pin 8. The other end of second lever 9 is pivotally connected to a third pin 11 parallel to pins 8 and 10 and mounted for slidably translating along an axial slot 12 formed in the seat 7 of the capsule body 1. The third pin 11 has enlarged guide ends 11 a for sliding on the side edges of the slot 12 and preventing the pin to escape from slot 12.

A pair of arms 13 and 14 are pivotaly connected to third pin 11 at one of their ends. Arms 13 and 14 lie on planes parallel to the lying planes of first and second levers 6 and 9 and extend in directions symmetrically inclined with respect to the longitudinal axis A of the capsule. The other ends of arm 13 and 14 are pivotally connected to respective hook means 15 and 16 each comprising a retractable hook portion 15a and 16a projecting from diametrically opposed slots 17 and 18 formed on front portion 3 of capsule body 1. Hook means 15 and 16 are formed by two coplanar plates 15b and 16b pivotally connected to seat 7 of the capsule body 1 through respective fourth pins 19 and 20, parallel to third pin 11, and having respective fifth pins 21 and 22 fixedly extending from plates 15b and 16b to pivotally connect these plates to arms 13 and 14 respectively. Fifth pins 21 and 22 are placed at a substantially diametrically opposed sides of plates 15b and 16b relative to fourth pins 19 and 20.

A motor 23 is placed in a seat 24 formed in the capsule body 1 parallel to the longitudinal axis of the capsule. The shaft of the motor 23 is supported for rotation in a bearing 25 at one end of seat 24 and a cam 26 is mounted on shaft 25 in an axial position such that the cam is in close proximity to the connection between first and second levers 6 and 9, in particular in correspondence to second pin 10. The profile of cam 26 is such that its minimum radius is not greater than the distance of the axis of the motor shaft from the edge of levers 6,9, while its maximum diameter is greater than this distance.

When the clip 4 is mounted on the front portion 3 of capsule body 1, as shown in figure 3, the clip 4 forces against the hook portions 15a and 16a of the hook means 15 and 16 projecting from the surface of the front portion 3 of capsule body 1 due to the elastic force stored on the clip to keep it in an enlarged condition. The force exerted on the hook portions 15a, 16a causes a compression force on third pin 11, directed along the longitudinal axis A, which, when first and second levers 6,9 are axially aligned, is discharged on first pin 8 thus keeping the mechanism in a stable equilibrium condition and allowing the hook portions 15a, 16a to retain the clip on the front portion 3 of body capsule 1.

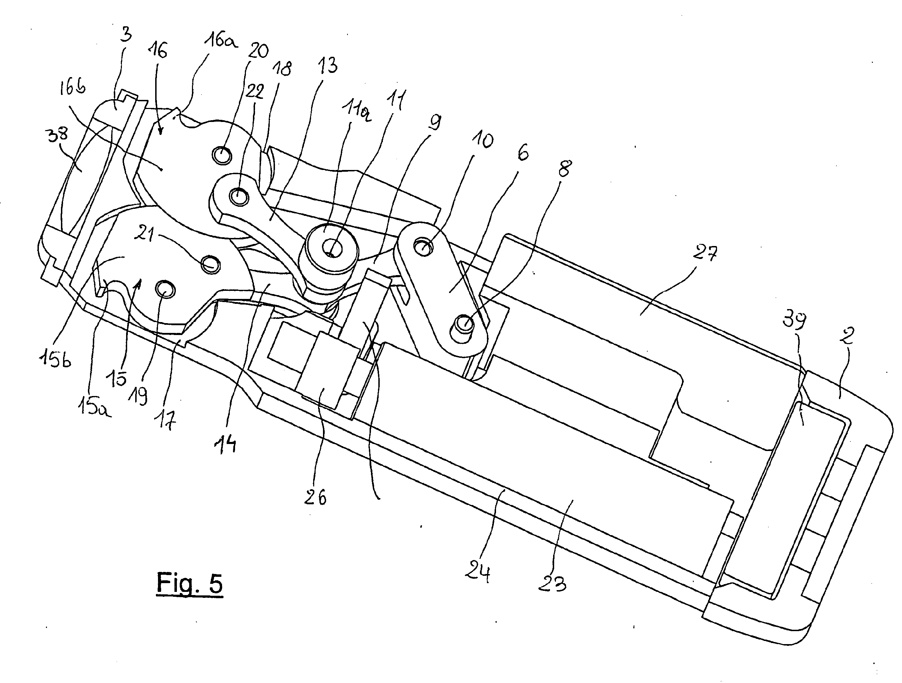

Upon actuation of the motor 23, a rotation of the motor shaft causes interference between cam 26 and levers 6,9. The pushing action exerted by the cam 26 unbalances the equilibrium condition of the mechanism, which immediately passes to the condition shown in figure 5. In fact, cam 26 urges the edges of levers 6,9 with its larger profile section thus displacing sidewards second pin 10 and causing levers 6,9 to rotate on their respective pins 8 and 11. The misalignment of first and second levers 6 and 9 causes the sliding of third pin 11 along axial slot 12, this resulting in opposite angular displacements of plates 15b, 16b and the consequent retraction of the hook portions 15a, 16a. At this point the clip, being no longer retained by the hook portions, is fired towards the target site by the action of the elastic force stored therewith.

Advantageously, to better distribute the force exerted by the cam 26 on levers 6, 9, a flexible rod 37 is provided between the cam 26 and the lever 6. The rod 37 extends from the seat 7 and is flexibly pushed against lever 6, 9 by the rotating cam.

The capsule is equipped with an on-board power source 27, such as a battery or a wireless energy supply system, to provide energy to the motor and to an on-board electronic control unit 39 and a video and imaging unit 38. In particular, the on-board electronic control unit is designed to transmit/receive commands or requests, transmit videos and/or images, acquiring data from sensors possibly equipped with the capsule. The control unit is equipped with microcontroller, wireless transmitter / receiver and high current drivers for actuator. A controller is also provided for the video and imaging unit. The battery can be of the Lithium Polymer rechargeable type and a wireless battery charger unit is provided.

The actuator can be a miniaturized DC brushless motor, controlled by a full bridge CMOS driver. The driver should be properly connected to the microcontroller, which is also in charge to wirelessly communicate with an external unit thorugh a dedicated wireless transceiver. A CMOS or CCD imaging sensor is used to acquire real time images, together with a proper illumination module. The image data stream is sent to the external unit by the telemetric link.

External facilities include a wireless dongle unit and a wireless video receiver unit and allow the collected data to be sent to a personal computer or a laptop for further processing. In particular, a wireless transceiver can be used for a bidirectional communication with the microcontroller inside the capsule and to receive the real time video streaming. The transceiver would be connected to a personal computer by a dedicate port, e.g. USB port. An user interface is implemented on the PC in order to properly display the image data and information related to the capsule operative status (e.g. battery level, telemetry signal strength, etc.). The user interface would also allow the user to fire the clip by pushing a dedicate button located on visual display.

Possible strategies for capsule locomotion, orientation and steering range from internal to external solutions. In particular, internal strategies may be implemented by placing a further actuator on board the capsule, as in WO 2008 122997. Having regard to the intended uses of the present invention, external strategies may prove to be more efficient. An example is external magnetic locomotion, where several small permanent magnets are placed on board the capsule and an external magnet, either permanent or composed by coils, is used to steer the capsule under the user control.

A solution of this type is disclosed for example in WO05122866, wherein the capsule is equipped with permanent magnets or elecromagnets and magnetic forces are applied to them by means of an external permanent magnet which is moved by the physician. In the present embodiment of the invention four permanent magnets 28 are arranged in respective axial seats 29 formed on the outer surface of the capsule body 1. For a proper operation of the capsule the Gl cavity wherein the capsule has to be moved is blown with air or other suitable gas or gas mixture to stretch the cavity walls.

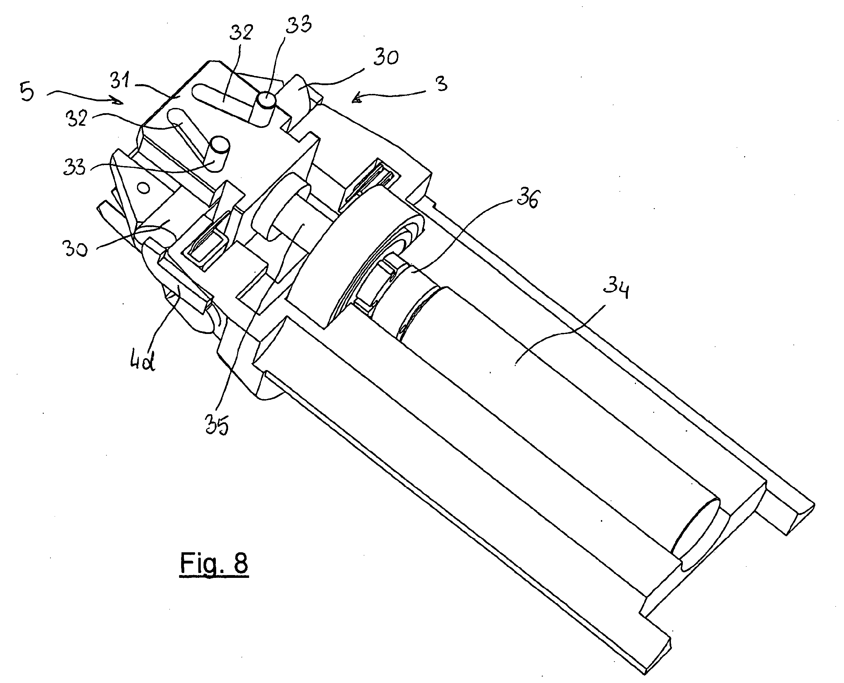

Figure 8 shows an alternative embodiment of clip releasing device for the wireless surgical clip delivering capsule according to the invention. In this embodiment the clip mounted on the front portion 3 of the capsule body 1 is passively released and the retaining action on the clip is provided by a pair of opposed stopper pins 30 diametrically projecting from the outer surface of the front portion 3 and operatively retractable therein as a result of an axial forward movement of a motorized slide 31 to which the pins 30 are connected. In particular, slide 31 is formed with two forwardly inclined slots 32 converging on the longitudinal axis A of the capsule, in which respective pegs 33 radially extending from stopper pins 30 are slidingly engaged. A motor 34 is axially housed in the capsule body 3 to rotate an axial screw 35 engaged with a nut screw (not shown) integral to slide 31. In this way a rotation of the screw 35 causes an axial movement of slide 31 and the retraction of pins 30. Preferably, a joint 36, such as a Oldahm joint, is provided between axial screw 35 and the motor shaft to reduce any placement error.

Preferably, the front portion 3 of capsule body 1 has a frusto-conical shape to make the clip release easier.

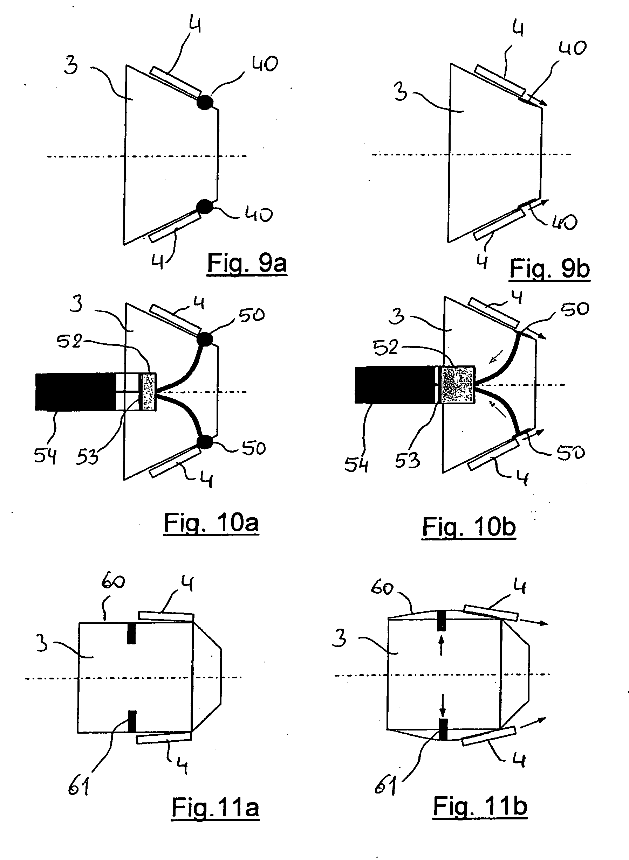

Figures 9a and 9b show a first variation of the clip releasing device of figure 8, in which the stopper pins 30 are replaced by small bodies such as spheres 40 projecting from the outer surface of front portion 3 of body capsule 1 to retain the clip 4. The spheres are made of SMA (Shape Memory Alloy) material and, when they are put under proper voltage, they collapse suddenly as shown in figure 9b causing the release of the clip 4.

Figures 10a and 10b show a second variation of the clip releasing device of figure 8, in which the stopper pins 30 are replaced by elastic bubbles 50 projecting from the front portion 3 of capsule body 1 to retain the clip 4 when they are full of a fluid. The bubbles 50 are hydraulically connected via tubes 51 to a chamber 52, whose volume can be varied by the movement of a piston 53 moved by an actuator 54. An increase of the volume of chamber 52 correspond to a suction action on the bubbles which collapse due to their emptying out as shown in figure 10b causing the release of the clip 4.

The advantages of embiodiments shown in figures 9a,b and 10a,b relative to embodiments shown in figures 3 to 8, may consist in that less mechanical components are required.

Figures 11 a and 11 b show a further embodiment of the clip releasing device, in which the clip is actively released from the capsule. In this case the front portion 3 of capsule body 1 on which the clip 4 is mounted has an outer surface 60 that can be raised ouwardly upon the action of a pushing member 61, which is retracted when the capsule is inoperative and projects outwards when the clip has to be released. The pushing member 61 is placed behind the clip 4, so that, when the outer surface 60 is raised, the supporting plane of the clip is inclined to cause it to be quickly released, as shown in figure 11 b. The pushing member 61 may be actuated by a mechanism such as that shown in figures 3 to 8.

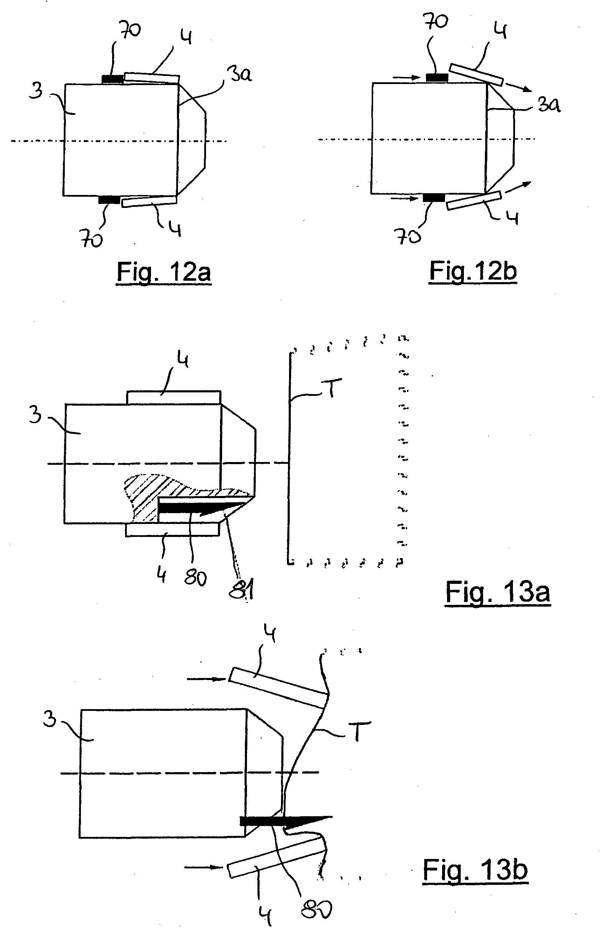

A variation of the above described active clip releasing device is shown in figures 12a and 12b, in which the clip 4 is pushed from a rest position shown in figure 12a to slide on the outer surface of front portion 3 of capsule body 1 until it reaches the front edge 3a of capsule body 1 and is released. To push the clip 4 at the back a pushing member 70, for example in the form of a coaxial sleeve or ring, can be provided and actuated by an axially moving mechanism, inside the capsule, radially connected to the pushing member.

In order to make easier the grasping action of the clip it may be advantageous to provide the capsule, at its front end, with tissue manipulation means for holding or withdrawing the tissue to be grasped by the clip before the clip is fired and in particular for moving the tissue close to the front end of the capsule before the clip is released. To this end it may be useful to equip the front end of the capsule with a sucker or an adhesive plate or hook/screw means for securing the capsule to the target site for a short time before the clip is released.

Figures 13a and 13b show an embodiment of the invention in which the capsule is equipped with an hook 80 housed in a seat 81 formed sideways at the front end 3 and parallel to the longitudinal axis A. Once the capsule reaches the target site the hook 80 is fired against the tissue T by an actuator, not shown, to get its tip stuck in the tissue. Then the capsule or the hook slightly goes back to draw out a tissue portion while the clip is released as shown in figure 13b. Once the clip grasps the tissue, the hook is released from the capsule. The use of a hook as aid for the grasping action may also assist in stabilizing the position of the capsule relative to the tissue when the clip is fired.

The capsule can be of the swallowable type and can be advantageously coated with a biocompatible and biodegradable layer that makes the swallowing action easier and safer. When the capsule reaches the stomach, the coating is destroyed by the acidity of the environment thus allowing it to become operative.

The wireless capsule for delivering a surgical clip according to the present invention allows a surgical clip, such that disclosed in US6428548, to be released by a wireless device instead of a flexible and tubular endoscope. The clip may be dispensed by a swallowable small pill dramatically reducing the pain and disconfort for the patient and increasing the number of potential candidates for this procedure. Furthermore, according to the present invention it will be possible to release the clip in areas of the gastrointestinal tract, such as the small bowel, that are unreachable and/or out of the operative field for most of the prior art endoscopic devices. This may open the way to innovative therapeutical procedures that were impossible before.

A kind of novel surgical procedure that can take tremendous advantage by this invention is the Natural Orifice Transluminal Endoscopic Surgery (NOTES). This is an experimental surgery technique wherein "scarless" abdominal operations can be performed with an endoscope passed through a natural body lumen (mouth, uretra, anus, etc.) then through an internal incision in the stomach, vagina, bladder or colon, thus avoiding any external incision or scars. Using a wireless device instead of a flexible endoscope may dramatically improve the efficiency and the results of this new technique. Another example of application is in the Bleeding Peptic Ulcer Disease, which can be a critical event owing to the fact that there is an internal haemorrhage associated with the ulcer. Another field of application of the capsule according to the present invention may be when a support for operating a surgical instrument or device has to be created in a body cavity or organ, in particular of the Gl tract. In this case the capsule of the present invention may be used to secure the clip serving as a support in the selected position.

The capsule according to the invention can be modified and varied in several ways, all of which are within the scope of the invention. All the details may further be replaced with other technically equivalent elements. In practice, the materials used, so long as they are compatible with the specific use, as well as the dimensions, may be any according to the requirements and the state of the art.

Wherever the characteristics and techniques described in any claim are followed by a specific reference, these have been included as an example for the sole purpose of making the claim descriptions easier to understand, and therefore they impose no limits on the interpretation of the element they refer to.

## Claims

1. A wireless capsule for delivering a surgical clip to a target site in the gastrointestinal (GI) tract of a patient comprising:
- a capsule body (1) with a front portion (3);
- a surgical clip (4) releasably mounted on the outer surface of the front portion of said capsule body;
- wireless locomotion and steering means (28) mounted on said capsule body;
- image sensing means 38 mounted on said capsule body for taking images of the GI tract and assisting in leading the capsule up to the target site;
- a transceiver unit 39 for transmitting the detected images to a data processing unit external to the patient and for receiving control signals from an operator;
- clip retaining and releasing means (5) for retaining the clip on the outer surface of said front portion of the capsule body when the capsule is moved through the GI tract, said clip retaining and releasing means being located on the outer surface of the front portion of the capsule body and being wirelessly actuable of for releasing the of when the capsule is in close proximity of the target surgical site and is directed towards said site.

2. The wireless capsule according to claim 1, wherein said clip retaining and releasing means comprise retractable stoppers (15,16,30,40,50) projecting from the outer surface of the front portion of said capsule body before said clip (4), which forcibly abuts against said stoppers, a motorized release mechanism (5) being provided inside said capsule body for retracting said stoppers, whereby the clip is released to grasp the tissue at the target site axially facing the capsule.

3. The wireless capsule according to claim 2, wherein said release mechanism comprise a slider (11,31) and a motor (23,34) for axially moving said slider, the slider being connected to said stoppers (15,16,30) in such a way that its axial sliding causes the stoppers to retract from said outer surface.

4. The wireless capsule according to claim 3, wherein said stoppers are in the form of substantially hook-shaped expansions (15a,16a) of at least a pair of plates (15b,16b) pivotally mounted within the front portion of said capsule body, said plates being pivotally connected to respective arms (13,14) at a connection point (21,22) placed at a diametrically opposite side of said expansions, said slider (11) being pivotally connected to said arms (13,14) and to a second lever (9), which, in turn, is pivotally connected to a first lever (6) pivotally connected to a pin (8) fixed to said capsule body and perpendicular to the longitudinal axis (A) of the capsule, said first and second levers (6,9) being axially aligned when said hook-shaped expansions (15a,16a) project from the outer surface of the front portion of the capsule body, a cam (26) driven by said motor being provided at a side of said first and second lever, the cam profile being such that an angular displacement of the cam causes a pushing force to be exerted on said levers in a direction perpendicular to their common pivotal axis, whereby the first and second levers are misaligned and the slider is moved along said longitudinal axis (A), thus retracting said hook-shaped expansions.

5. The wireless capsule according to claim 4, wherein the cam (26) is placed close to the common pivotal axis of said first and second lever (6,9).

6. The wireless capsule according to claim 3, wherein said stoppers comprise a pair of pins (30) extending at diametrically opposed sides of the front portion of said capsule body, said slider being a plate (31) substantially parallel to said pins and being formed with two symmetrically inclined slots (32) relative to the longitudinal axis (A) of the capsule slidably engaged whith respective feet (33) of said pins, a screw transmission (35) being provided between said motor (34) and said slider (31), whereby an axial angular displacement of the screw causes an axial movement of said slider, thus retracting said pins.

7. The wireless capsule according to claim 1, wherein said clip retaining and releasing means comprise collapsible stoppers (40,50) projecting from the outer surface of the front portion of said capsule body before said clip, which forcibly abuts against said stoppers, means being provided inside said capsule body for collapsing the stoppers, in such a way that the clip is released to grasp the tissue at the target site axially facing the capsule.

8. The wireless capsule according to claim 7, wherein said collapsible stoppers comprise at least a pair of shape memory alloy (SMA) bodies (40) and said collapsing means are an electric energy supplying circuit connected to said SMA bodies, whereby, when a voltage is applied to said SMA bodies, a sudden size reduction of the SMA bodies occurs and the clip is released.

9. The wireless capsule according to claim 7, wherein said collapsible stoppers comprise at least a pair of bubbles (50) full of a fluid and hydraulically connected to a chamber (52), whose volume can be varied by the movement of a piston (53) moved by an actuator (54), whereby an increase of the volume of said chamber corresponds to a suction action on said bubbles which collapse due to their emptying out, thus causing the release of the clip.

10. The wireless capsule according to claim 1, wherein said clip retaining and releasing means comprise pushing means (60,70,80)for axially sliding the clip on the outer surface of the front portion of the capsule body until the front edge of said front portion is reached to release said clip.

11. The wireless capsule according to claim 10, wherein said pushing means comprise an outwardly expandable surface (60) of said front portion of capsule body and a pushing member (61) acting from inside on side surface for raising outwardly said surface, said pushing member being placed behind the clip, so that, when the outer surface (60) is raised, the supporting plane of the clip is inclined to cause the clip to be quickly released.

12. The wireless capsule according to claim 10, wherein said pushing means comprise a sleeve or ring (70) coaxially movable at the rear side of said clip on the outer surface of said front portion of capsule body, said sleeve or ring being moved by motor means placed on-board of said capsule.

13. The wireless capsule according to any one of the previous claims, further comprising tissue manipulation means for holding or withdrawing the tissue to be grasped by the clip before the clip is fired.

14. The wireless capsule according to claim 13, wherein said tissue manipulation means comprise a sucker or an adhesive plate or hook/screw means (80) for securing the capsule to the target site before the clip is released.

## Patentansprüche

1. Drahtlose Kapsel zur Abgabe einer chirurgischen Klammer an einem Zielort im gastrointestinalen (GI) Trakt eines Patienten, enthaltend:
- einen Kapselkörper (1) mit einem Frontteil (3);
- eine chirurgische Klammer (4), die lösbar an der Außenoberfläche des Frontteils des Kapselkörpers angebracht ist;
- drahtlose Fortbewegungs- und Steuerungseinrichtungen (28), die an dem Kapselkörper angebracht sind;
- Bilderfassungseinrichtungen (38), die an dem Kapselkörper angebracht sind, um Bilder von dem GI-Trakt aufzunehmen und beim Führen der Kapsel hin zu dem Zielort zu assistieren;
- eine Sende/Empfangseinheit (39) zum Übertragen der detektierten Bilder zu einer Datenverarbeitungseinheit außerhalb des Patienten und zum Empfangen von Steuersignalen von einem Bediener;
- Klammer-Rückhalte- und -Freigabeeinrichtungen (5) zum Zurückhalten der Klammer an der Außenoberfläche des Frontteils des Kapselkörpers, wenn die Kapsel durch den GI-Trakt bewegt wird, wobei die Klammer-Rückhalte- und -Freigabeeinrichtungen an der Außenoberfläche des Frontteils des Kapselkörpers liegen und drahtlos betätigbar sind, um die Klammer freizugeben, wenn die Kapsel in unmittelbarer Nähe des chirurgischen Zielortes ist und diesem Ort zugewandt ist.

2. Drahtlose Kapsel nach Anspruch 1, wobei die Klammer-Rückhalte- und -Freigabeeinrichtungen rückziehbare Stopper (15, 16, 30, 40, 50) enthalten, die von der Außenoberfläche des Frontteils des Kapselkörpers vor die Klammer (4) vorstehen, die zwangsweise gegen die Stopper anliegt, wobei ein motorisierter Freigabemechanismus (5) innerhalb des Kapselköpers vorgesehen ist, um die Stopper zurückzuziehen, wodurch die Klammer freigegeben wird, um das Gewebe an dem Zielort zu greifen, das axial der Kapsel zugewandt ist.

3. Drahtlose Kapsel nach Anspruch 2, wobei der Freigabemechanismus einen Schieber (11, 31) und einen Motor (23, 34) zum axialen Bewegen des Schiebers enthält, wobei der Schieber mit den Stoppern (15, 16, 30) in einer solchen Weise verbunden ist, dass seine axiale Verschiebung die Stopper veranlasst, sich von der Außenoberfläche zurückzuziehen.

4. Drahtlose Kapsel nach Anspruch 3, wobei die Stopper in der Form von im wesentlichen hakenförmigen Erweiterungen (15a, 16a) wenigstens eines Paares von Platten (15b, 16b) sind, die drehbar innerhalb des Frontteils des Kapselkörpers angebracht sind, wobei die Platten drehbar mit jeweiligen Armen (13, 14) an einem Verbindungspunkt (21, 22) verbunden sind, der an einer diametral entgegengesetzten Seite der Erweiterungen angeordnet ist, wobei die Schieber (11) drehbar mit den Armen (13, 14) und mit einem zweiten Hebel (9) verbunden sind, der wiederum drehbar mit einem ersten Hebel (6) verbunden ist, der drehbar mit einem Stift (8) verbunden ist, der an dem Kapselkörper befestigt und senkrecht zur Längsachse (A) der Kapsel ist, wobei die ersten und zweiten Hebel (6, 9) axial ausgerichtet sind, wenn die hakenförmigen Erweiterungen (15a, 16a) von der Außenoberfläche des Frontteils des Kapselkörpers vorstehen, wobei ein Nocken (26), der von dem Motor angetrieben wird, an einer Seite des ersten und zweiten Hebels vorgesehen ist, wobei das Nockenprofil derart ist, dass ein winkelmäßiges Verstellen des Nockens verursacht, dass eine Schubkraft auf die Hebel in einer Richtung senkrecht zu ihrer gemeinsamen Drehachse ausgeübt wird, wodurch die ersten und zweiten Hebel unausgerichtet werden und der Schieber längs seiner Längsachse (A) bewegt wird, womit die hakenförmigen Erweiterungen zurückgezogen werden.

5. Drahtlose Kapsel nach Anspruch 4, wobei der Nocken (26) nahe der gemeinsamen Drehachse des ersten und zweiten Hebels (6, 9) angeordnet ist.

6. Drahtlose Kapsel nach Anspruch 3, wobei die Stopper ein Paar von Stiften (30) enthalten, die sich von diametral entgegengesetzten Seiten des Frontteils des Kapselkörpers aus erstrecken, wobei der Schieber eine Platte (31) ist, die im Wesentlichen parallel zu den Stiften ist und mit zwei symmetrisch relativ zu der Längsachse (A) der Kapsel geneigten Schlitzen (32) ausgebildet ist, in die jeweilige Füße (33) der Stifte verschiebbar eingreifen, wobei eine Gewindetransmission (35) zwischen dem Motor (34) und dem Schieber (31) vorgesehen ist, wodurch eine axiale winkelmäßige Verstellung der Schraube eine axiale Bewegung des Schiebers verursacht, womit die Stifte zurückgezogen werden.

7. Drahtlose Kapsel nach Anspruch 1, wobei die Klammer-Rückhalte- und -Freigabeeinrichtungen kollabierbare Stopper (40, 50) enthalten, die von der Außenoberfläche des Frontteils des Kapselkörpers vor die Klammer vorstehen, die zwangsweise gegen die Stopper anliegt, wobei Einrichtungen innerhalb des Kapselkörpers zum Kollabieren der Stopper in einer solchen Weise vorgesehen sind, dass die Klammer freigegeben wird, um das Gewebe an dem Zielort zu greifen, das axial der Kapsel zugewandt ist.

8. Drahtlose Kapsel nach Anspruch 7, wobei die kollabierbaren Stopper wenigstens ein Paar von Formgedächtnislegierung-(SMA) Körpern (40) enthalten und die kollabierenden Einrichtungen eine elektrische Energie zuführende Schaltung sind, die an die SMA-Körper angeschlossen ist, wodurch, wenn eine Spannung an die SMA-Körper angelegt wird, eine plötzliche Größenreduzierung der SMA-Körper auftritt und die Klammer freigegeben wird.

9. Drahtlose Kapsel nach Anspruch 7, wobei die kollabierbaren Stopper wenigstens ein Paar von Blasen (50) enthalten, die mit einem Fluid gefüllt und hydraulisch mit einer Kammer (52) verbunden sind, deren Volumen durch die Bewegung eines Kolbens (53) variiert werden kann, der durch einen Aktuator (54) bewegt werden kann, wodurch eine Vergrößerung des Volumens der Kammer einer Saugwirkung an den Blasen entspricht, die aufgrund ihrer Leerung kollabieren, womit das Freigeben der Klammer verursacht wird.

10. Drahtlose Kapsel nach Anspruch 1, wobei die Klammer-Rückhalte- und -Freigabeeinrichtungen Drückeinrichtungen (60, 70, 80) zum axialen Verschieben der Klammer an der Außenoberfläche des Frontteils des Kapselkörpers bis zum Erreichen des vorderen Randes des Frontteils enthalten, um die Klammer freizugeben.

11. Drahtlose Kapsel nach Anspruch 10, wobei die Drückeinrichtungen eine auswärts ausdehnbare Oberfläche (60) des Frontteils des Kapselkörpers und ein Drückelement (61) enthalten, das von innerhalb auf eine Seitenoberfläche wirkt, um die Oberfläche auswärts anzuheben, wobei das Drückelement hinter der Klammer angeordnet ist, so dass, wenn die Außenoberfläche (60) angehoben wird, die Stützebene der Klammer geneigt wird, um zu verursachen, dass die Klammer schnell freigegeben wird.

12. Drahtlose Kapsel nach Anspruch 10, wobei die Drückeinrichtungen eine Hülse oder einen Ring (70) enthalten, die/der koaxial an der hinteren Seite der Klammer an der Außenoberfläche des Frontteils des Kapselkörpers koaxial beweglich ist, wobei die Hülse oder der Ring durch Motoreinrichtungen bewegt wird, die an Bord der Kapsel angeordnet sind.

13. Drahtlose Kapsel nach einem der vorhergehenden Ansprüche, ferner enthaltend Gewebemanipulationseinrichtungen zum Halten oder Herausziehen des Gewebes, das von der Klammer gegriffen werden soll, bevor die Klammer abgefeuert wird.

14. Drahtlose Kapsel nach Anspruch 13, wobei die Gewebemanipulationseinrichtungen einen Sauger oder eine adhäsive Platte oder Haken-/Schraubeneinrichtungen (80) zum Sichern der Kapsel an dem Zielort enthalten, bevor die Klammer freigegeben wird.

## Revendications

1. Une capsule sans fil pour délivrer une pince chirurgicale vers une cible dans l'appareil gastro-intestinal (GI) d'un patient comprenant :
- un corps de capsule (1) avec une partie avant (3) ;
- une pince chirurgicale (4) apte à être libérée, montée sur la surface extérieure de la partie avant du dit corps de capsule ;
- des moyens de déplacement et de guidage sans fil (28) montés sur ledit corps de capsule ;
- des moyens de capture d'images (38) montés sur ledit corps de capsule pour prendre des images de l'appareil GI et pour aider à la conduite de la capsule jusqu'à la cible ;
- une unité de transmission (39) pour transmettre les images capturées vers une unité de traitement de données située à l'extérieur du patient et pour recevoir des signaux de commande émis par un opérateur ;
- des moyens de maintien en place et de libération (5) pour retenir la pince sur la surface extérieure de ladite partie avant du corps de capsule quand la capsule est déplacée dans l'appareil GI, lesdits moyens de maintien en place et de libération de la pince étant situés sur la surface extérieure de la partie avant du corps de capsule et étant aptes à être actionnés sans fil pour libérer la pince quand la capsule est à proximité de la cible chirurgicale et est dirigée vers ladite cible.

2. La capsule sans fil selon la revendication 1, où lesdits moyens (5) de maintien en place et de libération de la pince comportent des butées rétractables (15, 16, 30, 40, 50) s'étendant à partir de la surface extérieure de la partie avant du dit corps de capsule en avant de ladite pince (4), qui vient buter en force contre lesdites butées, un mécanisme de libération motorisé (5) étant agencé à l'intérieur du dit corps de capsule pour rétracter lesdites butées, ce en quoi la pince est libérée pour saisir le tissu de la cible faisant axialement face à la capsule.

3. La capsule sans fil selon la revendication 2, où ledit le mécanisme de libération comporte un curseur (11, 31) et un moteur (23, 34) pour déplacer axialement ledit curseur, le curseur étant connecté aux dites butées (15, 16, 30) de façon que son glissement axial fasse se rétracter les butées à partir de ladite surface extérieure.

4. La capsule sans fil selon la revendication 3, où lesdites butées sont des extensions en forme essentiellement de crochets (15a, 16a) d'au moins une paire de plaques (15b, 16b) montées pivotantes dans la partie avant du dit corps de capsule, lesdites plaques étant reliées de façon pivotante à des bras respectifs (13, 14) en un point de liaison (21, 22) diamétralement opposé aux dites extensions, ledit curseur (11) étant relié de façon pivotante aux dits bras (13, 14) et à un second levier (9), qui, à son tour, est relié de façon pivotante à un premier levier (6) relié de façon pivotante à un ergot (8) fixé au dit corps de capsule et perpendiculaire à l'axe longitudinal (A) de la capsule, lesdits premier et second leviers (6, 9) étant alignés axialement quand lesdites extensions en forme de crochets (15a, 16a) s'étendent à partir de la surface extérieure de la partie avant du corps de capsule, une came (26) pilotée par ledit moteur étant agencée sur un côté des dits premier et second leviers, le profil de la came étant tel qu'un déplacement angulaire de la came entraîne une force de poussée exercée sur lesdits leviers dans une direction perpendiculaire à leur axe commun de pivotement, ce en quoi les premier et second leviers sont désalignés et le curseur est déplacé le long du dit axe longitudinal (A), rétractant ainsi lesdites extensions en forme de crochets.

5. La capsule sans fil selon la revendication 4, où la came (26) est placée à proximité de l'axe commun de pivotement des dits premier et second leviers (6,9).

6. La capsule sans fil selon la revendication 3, où lesdites butées comportent une paire de doigts (30) s'étendant sur les côtés diamétralement opposés de la partie avant du dit corps de capsule, ledit curseur étant une plaque (31) sensiblement parallèle aux dits doigts et comportant deux fentes symétriquement inclinées (32) par rapport à l'axe longitudinal (A) de la capsule coopérant de façon coulissante avec les bases respectives (33) des dits doigts, une vis de transmission (35) étant agencée entre ledit moteur (34) et ledit curseur (31), ce en quoi un déplacement angulaire axial de la vis entraîne un mouvement axial du dit curseur, faisant ainsi se rétracter lesdits doigts.

7. La capsule sans fil selon la revendication 1, où lesdits moyens de maintien en place et de libération de la pince comportent des butées escamotables (40, 50) s'étendant à partir de la surface extérieure de la partie avant du dit corps de capsule en avant de ladite pince, qui bute en force contre lesdites butées, des moyens étant agencés à l'intérieur du dit corps de capsule pour escamoter les butées, de façon que la pince soit libérée pour saisir le tissu de la cible faisant axialement face à la capsule.

8. La capsule sans fil selon la revendication 7, où lesdites butées escamotables comportent au moins une paire de corps (40) en alliage à mémoire de forme (SMA) et où lesdits moyens pour les escamoter sont un circuit d'alimentation en énergie électrique relié aux dits corps en SMA, ce en quoi, quand une tension est appliquée aux dits corps en SMA, une réduction soudaine de taille des corps en SMA se produit et la pince est libérée.

9. La capsule sans fil selon la revendication 7, où lesdites butées escamotables comportent au moins une paire de ballonnets (50) remplis d'un fluide et reliés hydrauliquement à une chambre (52), dont le volume peut être modulé par le mouvement d'un piston (53) déplacé par un vérin (54), ce en quoi une augmentation du volume de ladite chambre correspond à une action d'aspiration dans lesdits ballonnets qui se dégonflent en raison du vide qui y est ainsi réalisé, entraînant de ce fait la libération de la pince.

10. La capsule sans fil selon la revendication 1, où lesdits moyens de maintien en place et de libération de la pince comportent des moyens de poussée (60, 70, 80) pour faire glisser axialement la pince sur la surface extérieure de la partie avant du corps de capsule jusqu'à ce que le bord avant de ladite partie avant soit atteint pour libérer ladite pince.

11. La capsule sans fil selon la revendication 10, où lesdits moyens de poussée comportent une surface (60) expansible vers l'extérieur de ladite partie avant du corps de capsule et un élément de poussée (61) agissant par l'intérieur sur la surface latérale pour soulever ladite surface vers l'extérieur, ledit élément de poussée étant positionné derrière la pince, ce en quoi, quand la surface extérieure (60) est soulevée, le plan supportant la pince est incliné et en conséquence la pince est rapidement libérée.

12. La capsule sans fil selon la revendication 10, où lesdits moyens de poussée comportent un manchon ou un anneau (70) mobile de façon coaxiale à l'arrière de ladite pince sur la surface extérieure de ladite partie avant du corps de capsule, ledit manchon ou anneau étant déplacé par des moyens moteurs situés dans ladite capsule.

13. La capsule sans fil selon l'une quelconque des revendications précédentes, comprenant en outre des moyens de manipulation de tissu pour maintenir ou retirer le tissu qui doit être saisi par le pince avant que la pince soit mise en action.

14. La capsule sans fil selon la revendication 13, où lesdits moyens de manipulation de tissu comportent une ventouse ou une plaque adhésive ou des moyens de vis/crochet (80) pour fixer la capsule sur la cible avant que la pince soit libérée.
